# FASCICULE DE BREVET EUROPEEN

(11) **EP 3 352 722 B1**
(45) Date de publication et mention de la délivrance du brevet: **07.08.2019**
(21) Numéro de dépôt: 16781508.3
(22) Date de dépôt: 19.09.2016
(51) Int. Cl.: A61H 7/00, A61N 5/00, A61H 15/02, A61H 15/00

(54) **APPAREIL DE SOIN AVEC GUIDE DE LUMIÈRE**
BEHANDLUNGSVORRICHTUNG MIT LICHTLEITER
TREATMENT DEVICE WITH LIGHT GUIDE

(30) Priorité: 21.09.2015 FR 1558888
(43) Date de publication de la demande: 01.08.2018
(73) Titulaire: SEB S.A., 69130 Ecully (FR)
(72) Inventeur: SABATTIER, Johan, 69440 Mornant (FR); MANDICA, Franck, 69340 Francheville (FR)
(74) Mandataire: Bourrières, Patrice
(86) Numéro de dépôt international: PCT/FR2016/052368
(87) Numéro de publication internationale: WO 2017/051102

(56) Documents cités:
- EP-A1- 2 862 555
- EP-A1- 2 862 556
- US-A1- 2009 177 125
- US-A1- 2014 135 798

## Description

La présente invention concerne un appareil de soin de la peau, en particulier de la peau du visage, destiné à fournir des soins sous formes variées pour produire un effet coup d'éclat, remodelage, anti-âge ou antirides. Les soins sont procurés par un apport d'énergie lumineuse à la peau et également par une action mécanique de moyens massants.

On connaît un document KR20090001911 qui enseigne un appareil de massage comprenant une tête de soin comprenant une chambre sous dépression destinée à aspirer la peau. La chambre intègre deux rouleaux parallèles qui sont motorisés de sorte à tourner dans le même sens. L'appareil comprend encore un émetteur de lumière dans le fond de la chambre derrière lesdits rouleaux. La source lumineuse est du type diode électroluminescente, communément appelée LED, destinée à projeter de la lumière simultanément au mouvement des rouleaux. L'inconvénient d'un tel agencement de la source lumineuse est qu'elle est éloignée de la zone à projeter, c'est-à-dire du plan tangent à l'extérieur des rouleaux, ce qui fait que l'intensité de la lumière projetée est diminuée. De plus, une partie des faisceaux lumineux est dissimulée par les deux rouleaux, ce qui réduit l'efficacité du traitement par la lumière.

On connaît un autre document CN202537881 qui décrit un appareil de massage comprenant une tête de soin présentant des têtes de massage destinées à masser la peau. Les têtes sont motorisées pour effectuer un mouvement en rotation. L'appareil comprend en outre des sources lumineuses agencées à l'intérieur de la tête de soin afin d'apporter un traitement autre que le massage mécanique. Cependant, cet appareil a pour inconvénient une projection de lumière peu puissante et surtout peu homogène, que ce soit sur les têtes de massage ou dans la zone qui se trouve entre les têtes.

Le document EP2862556 décrit le préambule de la revendication 1.

Le but de la présente invention est de remédier au moins en partie aux inconvénients précités et de proposer un appareil de soin de la peau permettant de fournir au moins un traitement mécanique combiné à un traitement par lumière sur une zone définie.

Un autre but de l'invention est un appareil de soin de la peau capable de produire un éclairage à intensité ciblée et homogène sur une surface définie.

Un autre but de l'invention est un appareil de soin de la peau dont les sources lumineuses permettent d'éclairer au moins une partie d'une zone sollicitée par des moyens de massage mécaniques auxquels elles sont associées.

Un autre but de l'invention est un appareil de soin de la peau avec un agencement des sources lumineuses et des moyens de massage mécaniques optimisé pour un encombrement de l'appareil réduit.

Encore un autre but de l'invention est un appareil de soin de la peau multifonctionnel, facile à utiliser et peu coûteux.

Ces buts sont atteints avec un appareil de soin pour la peau comprenant :
- un corps comportant un boîtier formant une zone de préhension,
- des moyens d'alimentation électrique agencés dans ledit corps,
- au moins une tête de soin comprenant des moyens de massage,
- des moyens de manoeuvre desdits moyens de massage actionnés par un moteur électrique qui est relié auxdits moyens d'alimentation électrique,
- au moins une source lumineuse,
- au moins un guide de lumière présentant une surface de projection destinée à recevoir de la lumière émise par ladite au moins une source lumineuse et à projeter au moins un faisceau lumineux à destination d'une zone sur la peau.

Selon l'invention, ledit au moins un guide de lumière comprend une face opposée à ladite surface de projection, ladite face opposée comprenant des moyens de distribution destinés à distribuer la lumière le long de ladite surface de projection, et au moins un moyen de massage présente une zone (Z) de contact avec la peau à masser, ladite zone (Z) étant destinée à transmettre la lumière et étant constituée d'un matériau du type thermoplastique élastomère (TPE) présentant une transmission lumineuse comprise entre 0,8 et 0,9 pour une lumière ayant une longueur d'onde de 590nm ou 630nm, connue pour ses effets de photomodulation.

Ainsi, la lumière atteignant la face opposée est redirigée vers la surface de projection. La lumière diffusée par la surface de projection est ensuite projetée en direction de la peau mais également en direction de l'au moins un moyen de massage qui de par la constitution de sa zone en contact avec la peau à masser transmet également la lumière en direction de la peau au lieu de simplement l'occulter. Cela permet de diffuser davantage de lumière, de façon homogène et au plus près de la peau. Les effets de ces lumières connus sur la peau sont notamment la stimulation de production de collagène et plus généralement une amélioration de l'aspect de la peau pour lui donner un aspect plus jeune et plus homogène. Ces effets sont notamment décrits dans de nombreuses publications par Mc Daniels et Weiss depuis 2004. Le matériau présentant une telle propriété optique permet une meilleure transmission des lumières visibles ainsi que l'infrarouge.

Avantageusement, ledit matériau présente une dureté entre 30 et 70 Shore A. Ceci permet d'apporter une sensation agréable au toucher et de rendre le massage plus efficace.

De surcroît, ladite zone (Z) de contact présente un état de surface non poli avec une rugosité arithmétique Ra de l'ordre de 0,5µm à 1µm. Un tel état de surface permet d'améliorer l'uniformité du flux de lumière.

Selon l'invention, lesdits moyens de massage comprennent au moins une tête de massage destinée à être déplacée en rotation selon au moins un axe de rotation vertical (A1, A2, A3) qui est perpendiculaire à la surface de projection.

Avantageusement, ladite tête de massage présente une portion hémisphérique. La tête de massage a donc un bout arrondi permettant un massage agréable.

De surcroît, ladite tête de massage comprend une couverture définissant ladite zone (Z) de contact et ladite couverture est fixée à la portion hémisphérique par surmoulage. Ladite couverture est donc destinée à prendre appui sur la peau pendant le traitement. Elle est fabriquée de façon indépendante de la tête de massage de telle sorte que cette dernière peut être réalisée en une autre matière plus rigide pour assurer la solidité de l'appareil.

Selon une autre caractéristique de l'invention, ladite source lumineuse comprend une pluralité de diodes électroluminescentes réparties de façon régulière autour dudit guide de lumière. Ceci permet de bien éclairer la zone de traitement depuis toutes les directions.

Selon une autre caractéristique de l'invention, lesdits moyens de distribution comprennent au moins une pente périphérique destinée au renvoi de lumière vers la périphérie du guide de lumière. Ceci permet de bien éclairer la périphérie de la zone de traitement.

Selon encore une autre caractéristique de l'invention, lesdits moyens de distribution comprennent au moins une pente intermédiaire destinée au renvoi de lumière vers une zone intermédiaire du guide de lumière. Ceci permet de bien éclairer la zone entre les têtes de massage et la périphérie.

Selon encore une autre caractéristique de l'invention, ladite tête de massage comprend des moyens de réflexion permettant un renvoi de lumière vers ladite zone (Z) de contact. Ceci permet de faire passer la lumière dans la tête de massage et ensuite vers la zone de contact.

Avantageusement, ladite au moins une tête de soin est détachable dudit corps. Il est envisageable des moyens de fixation réversibles qui sont à la portée de l'homme du métier.

De surcroît, l'appareil comprend au moins deux têtes de soin différentes interchangeables sur le corps afin de proposer au moins deux traitements différents dans un même appareil aux utilisateurs.

L'invention sera mieux comprise à l'étude des modes de réalisation pris à titre nullement limitatif et illustrés par les dessins annexés dans lesquels :
- La figure 1 est une vue en perspective de l'appareil selon l'invention,
- La figure 2 est une vue en perspective de l'intérieur de l'appareil,
- Les figures 3 et 4 illustrent la tête de soin selon l'invention,
- La figure 5 est une vue en perspective du guide de lumière selon un premier mode de réalisation,
- Les figures 6 à 8 illustrent le guide de lumière selon un deuxième mode de réalisation,
- La figure 9 est une vue en éclaté d'un moyen massant,
- La figure 10 est une vue de coupe du guide de lumière selon le deuxième mode de réalisation,
- La figure 11 est une vue de coupe du moyen massant.

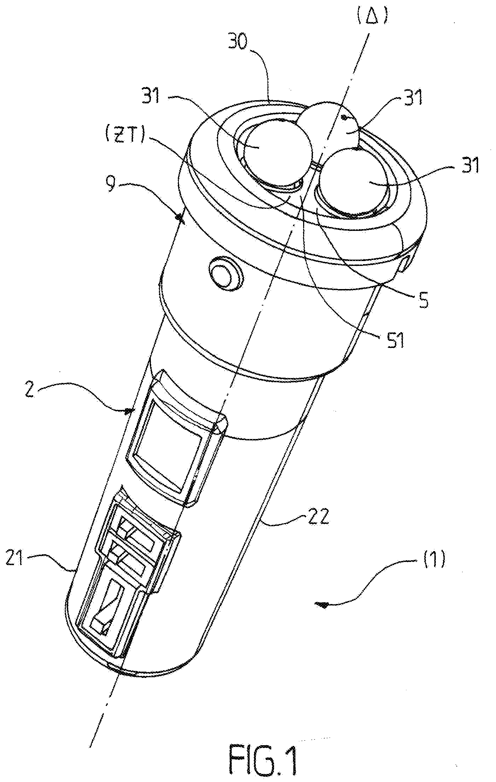

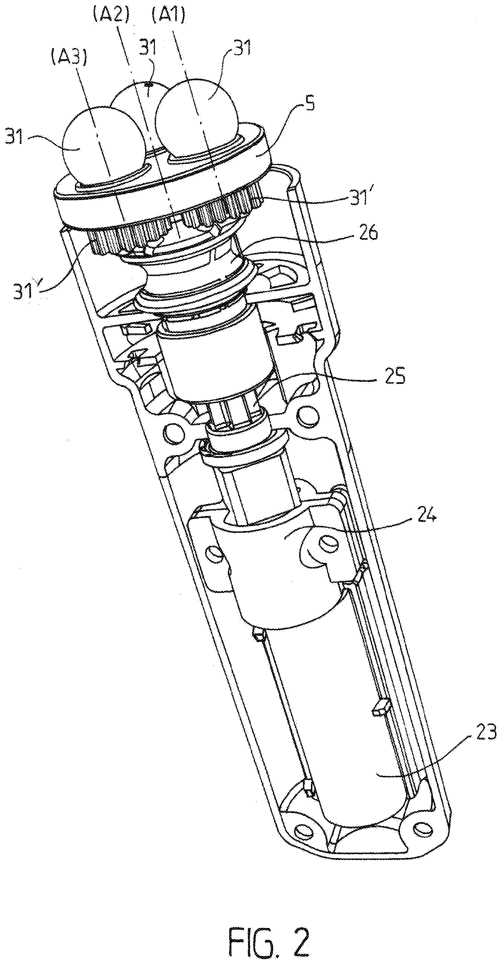

L'appareil de soin pour la peau tel qu'illustré aux figures 1 et 2 et désigné dans son ensemble par la référence 1 comprend une tête de soin 9 et un corps 2. Ledit corps 2 ayant une forme longitudinale et d'axe central (Δ) comprend un boîtier constitué de deux demi-boîtiers 21, 22 formant ensemble une zone de préhension. La tête de soin 9 étant montée sur une des extrémités du corps 2 présente une zone de traitement (ZT) destinée à fonctionner sur ou près de la peau. Ladite zone de traitement (ZT) est la combinaison d'une zone de projection de lumière et d'une zone présentant des moyens de massage mécanique La tête de soin 9 présente une forme sensiblement cylindrique coaxiale avec le corps 2 de l'appareil. La tête de soin 9 comprend une couronne 30 d'appui délimitant la zone de traitement (ZT) à l'intérieur de laquelle se trouvent des moyens de massage qui sont trois têtes de massage 31 s'étendant en saillie du plan de la couronne 30. La tête de soin 9 comprend des moyens d'adaptation sur le corps 2 de façon amovible, lesdits moyens d'adaptation pouvant être formés par un fourreau permettant à la tête de soin 9 d'être en partie engagée dans le corps 2. Ainsi, l'appareil peut comprendre plusieurs têtes de soin différentes à utiliser sur le corps 2 de façon interchangeable. Tel que visible à la figure 2, le corps 2 comprend un moteur électrique 24 relié à un arbre de sortie 25 par des moyens de transmission, l'arbre de sortie 25 étant accessible au niveau de l'extrémité des moyens d'adaptation. Le moteur électrique 24 est alimenté par des moyens d'alimentation électrique par exemple une batterie 23 et piloté par une unité de commande raccordée à une interface de commande manuelle accessible depuis l'extérieur du corps 2. L'interface de commande manuelle peut par exemple comprendre un interrupteur marche-arrêt et/ou des moyens de sélection manuelle de programmes de fonctionnement.

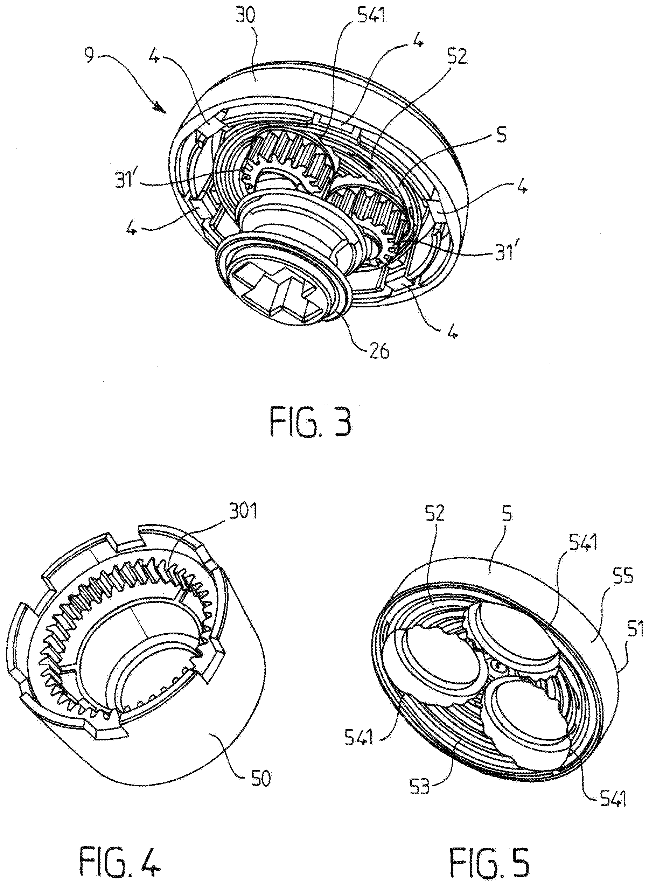

Tel qu'illustré aux figures 2 à 4, chaque tête de massage 31 est reliée à des moyens de manoeuvre 26, 31' pour être entrainée en rotation non seulement par rapport à l'axe central (Δ) mais aussi par rapport à un axe de rotation vertical A1, A2, A3 qui lui est propre. Pour ce faire, les moyens de manoeuvre 26, 31' comprennent un entraineur 26 destiné à s'emboîter avec l'arbre de sortie 25 du moteur pour sa mise en mouvement. Par exemple, l'entraineur 26 peut présenter une cavité en forme de croix destinée à recevoir l'arbre de sortie 25 par une protubérance de la même forme sur celui-ci. L'entraineur 26 d'axe (Δ) porte sur une face supérieure trois pions (non illustrés) engagés chacun dans un alésage axial d'un pignon planétaire 31'. Chaque tête de massage est fixée sur le pignon planétaire correspondant. Chaque pignon planétaire engrène la périphérie dentée 301 d'une bague fixe 50, illustrées à la figure 4, de sorte que la rotation de l'entraineur 26 entraine le mouvement planétaire des têtes de massage 31, c'est-à-dire un mouvement de rotation principal des trois têtes autour de l'axe central (Δ) combiné à un mouvement de rotation secondaire de chacune des têtes autour de l'axe de rotation vertical A1, A2, A3 qui se déplace en rotation autour de l'axe central (Δ) lors du fonctionnement de l'appareil.

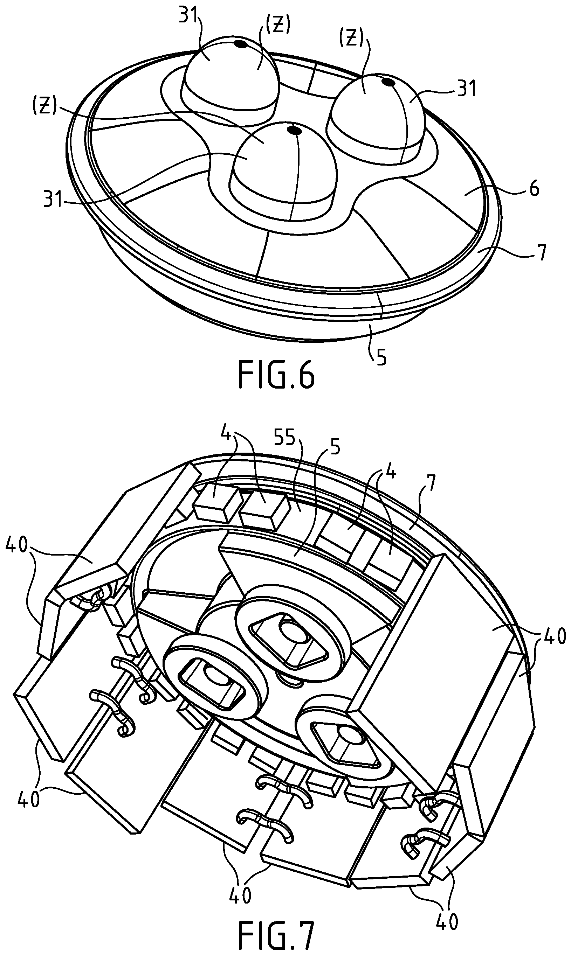

La tête de soin 9 telle que présentée permet de pétrir la peau du visage, notamment les zones larges telles que les joues ou le front, pour activer la microcirculation et stimuler les mécanismes naturels de production des protéines structurantes de la peau et ainsi prévenir les signes de l'âge. Ces effets sont accentués par la présence de la lumière sur la peau. Pour ceci, l'appareil comprend une pluralité de sources lumineuses 4 qui sont des LED (diodes électroluminescentes) agencées à l'intérieur de la tête de soin 9. Selon l'exemple illustré à la figure 7, les LED 4 sont au nombre de 24 et sont réparties de façon régulière sur le pourtour de la tête de soin 9. Des moyens de support 40 pour les LED peuvent être utilisés tel que des circuits imprimés (ou PCB de l'anglais *Printed Circuit Boards*) qui sont des plaques intégrant un circuit électrique permettant d'alimenter les LED qui sont montées dessus. Cette technique d'agencement de LED est connue pour l'homme du métier. Les PCB sont de préférence blancs pour optimiser l'émission de la lumière vers l'intérieur de l'appareil. Chaque PCB supporte deux LED de façon à avoir une distance entre chaque LED et le guide de lumière plus constante. L'ensemble des LED sont positionnées sur une même ligne de façon à diminuer l'influence de la rotation de la tête de soin. De préférence, 18 LED visibles et 6 LED Infrarouges sont utilisées.

Afin de bien répartir la lumière sur la zone de traitement (ZT), la tête de soin 9 comprend en son intérieur un guide de lumière 5 qui a pour objectif d'orienter et d'homogénéiser et de concentrer les faisceaux lumineux provenant des LED vers la peau. Pour ce faire, ledit guide de lumière 5 présente une forme de plateau sensiblement circulaire et monobloc ayant une surface extérieure lisse qui est une surface de projection 51 de la lumière. Le guide de lumière 5 présente encore une face opposée 52 qui est agencée en regard de ladite surface de projection 51 et écartée de celle-ci par une paroi latérale 55 sensiblement cylindrique. Les LED sont donc réparties autour du guide de lumière 5 face à la paroi latérale 55. Ladite face opposée 52 comprend des moyens de distribution 53 destinés à distribuer la lumière le long de la surface de projection 51.

Selon un premier mode de réalisation du guide de lumière 5 et tel que visible à la figure 5, les moyens de distribution 53 présentent une série de stries circulaires et concentriques qui sont inscrites sur la face opposée 52 qui est légèrement concave ou conique vers l'intérieur. Des trous traversants 541 permettent de laisser passer les têtes de massage 31. Le guide de lumière 5 est entrainé en rotation grâce au mouvement des têtes. D'autres formes de moyens de distribution pourraient également convenir pour guider la lumière telles que des cannelures inscrites sur la face opposée plate ou concave ou conique vers l'intérieur. On peut aussi combiner les stries et les cannelures pour avoir un effet de réflexion dans un périmètre plus large.

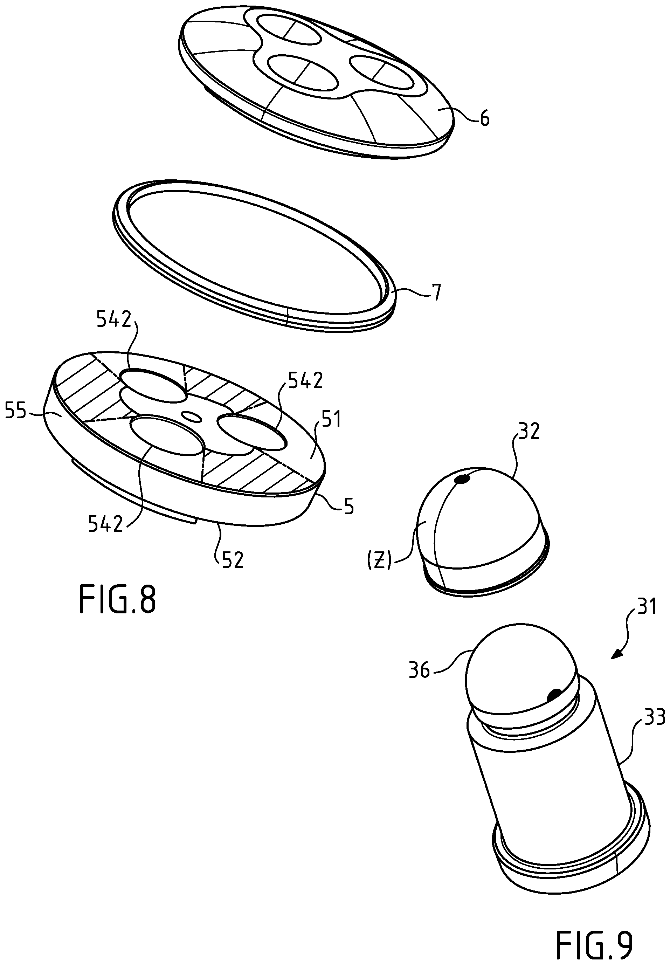

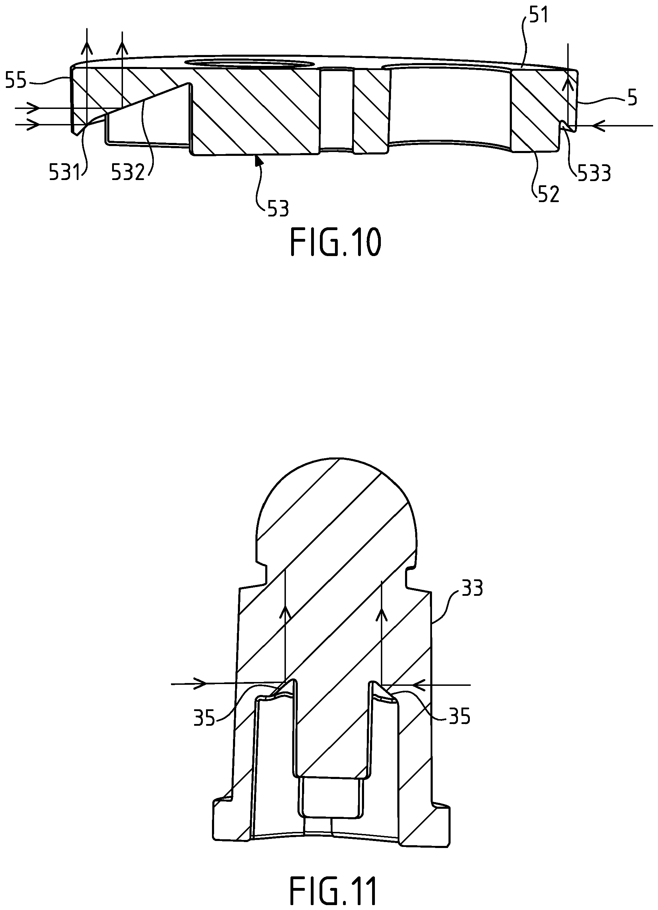

Selon un deuxième mode de réalisation du guide de lumière 5 et tel que visible aux figures 7, 8 et 10, les moyens de distribution 53 comprennent des pentes périphérique 531, 533 destinées au renvoi de lumière vers la périphérie du guide de lumière 5. La Figure 10 illustre une vue de coupe permettant de visualiser lesdites pentes périphériques 531, 533. Trois trous traversants 542 permettent de laisser passer les têtes de massage. Les moyens de distribution 53 comprennent encore des pentes intermédiaires 532 qui contribuent au renvoi de lumière vers une zone intermédiaire du guide qui correspond à une zone entre la périphérie et les trous traversants 542, cette zone est illustrée à la figure 8 par les hachures. Le fait que la lumière ne s'étende que sur trois portions angulaires permet d'optimiser la proportion de lumière réfléchie vers le centre de la zone de traitement (ZT) et la partie laissée disponible pour les têtes de massage. Plus les portions de guides sont grandes, plus on favorise le centre, plus elles sont petites, plus on favorise les têtes de massage.

Le guide de lumière 5 est de préférence en polyméthacrylate de méthyle (PMMA), matière qui présente un bon indice de milieu donc une bonne réflexion aux interfaces, et une bonne transparence donc peu d'absorption d'énergie. La méthode d'optimisation est relativement simple et basée sur le principe de réflexion totale interne. Lorsque la lumière est injectée dans un matériau et arrive sur une surface, si l'incidence est suffisamment importante, alors la totalité de cette lumière sera réfléchie et restera ainsi « piégée » par la matière. L'avantage de cette réflexion (et par opposition à une réflexion sur une surface métallisée) est qu'elle se fait sans perte et peut donc être répétée un grand nombre de fois.

Les LED 4 sont agencées de telle sorte que lors de la projection dans le guide de lumière 5, l'angle d'incidence soit supérieur à 42° qui est la valeur limite pour atteindre le maximum de réflexion dans du PMMA.

Afin d'améliorer l'uniformité de l'éclairement, il est envisageable d'utiliser une pièce supplémentaire qui est une vitre façade 6 par-dessus le guide de lumière 5. Tel que visible aux figures 6 et 8, cette pièce épouse la forme du guide de lumière 5 pour être collée contre celle-ci. Un anneau 7 permet de maintenir le tout en place. Ladite vitre façade 6 est faite d'un matériau spécifique qui est un polyméthacrylate de méthyle (PMMA) auquel ont été ajoutées des microbilles de verre. Ces microbilles se comportent comme des gouttelettes d'eau en suspension dans l'air, générant un brouillard. Le matériau a ainsi un aspect laiteux dû à la diffusion de la lumière dans la matière. La vitre façade 6 peut également présenter un état de surface de type charmille pour les faces inférieures et supérieures de la pièce. Il est possible de réaliser l'ensemble du guide de lumière 5 et la vitre façade 6 par un procédé de bi injection.

En ce qui concerne les moyens de massage 31, ils présentent chacun une zone (Z) de contact avec la peau à masser comme visible à la figure 6. Une vue en éclaté d'un moyen de massage 31 illustré à la figure 9 montre que le moyen de massage ou la tête de massage 31 présente une portion 36 hémisphérique laquelle est reliée à un corps cylindrique 33. La tête de massage 31 comprend en outre une couverture 32 définissant ladite zone de contact et qui se met par-dessus la portion 36. Ladite couverture 32 est faite d'un matériau du type thermoplastique élastomère (TPE) permettant une homogénéité d'éclairement. Plus particulièrement, ledit matériau présente une transmission lumineuse comprise entre 0,8 et 0,9 pour une lumière ayant une longueur d'ondes de 590nm ou de 630nm.

Les valeurs de transmission lumineuse peuvent être mesurées à l'aide d'un spectrophotomètre. L'échantillon à analyser est alors placé dans cet appareil de référence lors de la mesure.

En outre, ledit matériau présente une dureté entre 30 et 70 Shore A pour une finition agréable au toucher.

Le matériau peut être choisi dans la famille du copolymère bloc styrénique hydrogéné (HSBC). Certains matériaux de la famille du polyuréthane (PU) ou des copolymères éthylène-styrène/buthylène-styrène (SEBS) peuvent également convenir.

Il est préférable de choisir un matériau adapté pour l'appareil de soin décrit ici qui présente une bonne compatibilité avec les cosmétiques susceptibles d'être appliqués sur la peau durant l'utilisation de l'appareil, ainsi qu'une bonne adhésion sur le polyméthacrylate de méthyle (PMMA), matériau de la pièce sur laquelle il est surmoulé, par exemple, le matériau TEFI 910 de MITSUBISHI CHEMICAL.

Ladite couverture 32 est reliée à ladite portion 36 par surmoulage. Il est possible de faire sur la couverture 32 une zone de contact ayant un état de surface non poli avec une rugosité arithmétique Ra de l'ordre de 0,5µm à 1µm pour compenser un éventuel manque de diffusion volumique par de la diffusion surfacique.

Afin de mieux éclairer la zone de traitement (ZT), surtout les endroits où il y a les têtes de massage 31, chaque tête de massage 31 comprend en son intérieur des moyens de réflexion 35 permettant un renvoi de lumière vers ladite zone (Z) de contact. Comme illustré à la figure 11, la tête de massage présente une surface penchée 35 en face de la surface latérale du corps cylindrique 33 ce qui permet de renvoyer la lumière intégralement vers le haut du corps cylindrique 33.

Afin de masquer l'intérieur du guide de lumière et de rendre la projection du faisceau lumineux la plus homogène possible, la surface de projection 51 peut être dépolie ou grainée, par exemple avec un grainage de type chimique ou charmille.

Bien entendu, l'invention n'est nullement limitée aux modes de réalisation décrits et illustrés qui n'ont été donnés qu'à titre d'exemple. Des modifications restent possibles, notamment du point de vue de la constitution des divers éléments ou par substitution d'équivalents techniques, sans sortir pour autant du domaine de protection de l'invention, qui est défini par les revendications.

## Revendications

1. Appareil de soin (1) pour la peau comprenant :
- un corps (2) comportant un boîtier (21, 22) formant une zone de préhension,
- des moyens d'alimentation électrique (23) agencés dans ledit corps,
- au moins une tête de soin (9) comprenant des moyens de massage (31)
- des moyens de manoeuvre (26, 31') desdits moyens de massage (31) actionnés par un moteur électrique (24) qui est relié auxdits moyens d'alimentation électrique (23),
- au moins une source lumineuse (4),
- au moins un guide de lumière (5) présentant une surface de projection (51) destinée à recevoir de la lumière émise par ladite au moins une source lumineuse (4) et à projeter au moins un faisceau lumineux à destination d'une zone sur la peau,
**caractérisé en ce que** ledit au moins un guide de lumière (5) comprend une face opposée (52) à ladite surface de projection (51), ladite face opposée (52) comprenant des moyens de distribution (53) destinés à distribuer la lumière le long de ladite surface de projection (51), et **en ce qu'**au moins un moyen de massage (31) présente une zone (Z) de contact avec la peau à masser, ladite zone (Z) étant destinée à transmettre la lumière et étant constituée d'un matériau du type thermoplastique élastomère (TPE) présentant une transmission lumineuse comprise entre 0,8 et 0,9 pour une lumière ayant une longueur d'onde de 590nm ou 630nm.

2. Appareil selon la revendication 1, **caractérisé en ce que** ledit matériau présente une dureté entre 30 et 70 Shore A.

3. Appareil selon l'une des revendications précédentes, **caractérisé en ce que** ladite zone (Z) de contact présente un état de surface non poli avec une rugosité arithmétique Ra de l'ordre de 0,5µm à 1µm.

4. Appareil selon l'une des revendications précédentes **caractérisé en ce que** lesdits moyens de massage (31) comprennent au moins une tête de massage (31) destinée à être déplacée en rotation selon au moins un axe de rotation vertical (A1, A2, A3) qui est perpendiculaire à la surface de projection (51).

5. Appareil selon la revendication précédente, **caractérisé en ce que** ladite tête de massage (31) présente une portion (36) hémisphérique.

6. Appareil selon la revendication 4, **caractérisé en ce que** ladite tête de massage (31) comprend une couverture (32) définissant ladite zone (Z) de contact.

7. Appareil selon la revendication précédente, **caractérisé en ce que** ladite couverture (32) est fixée à la portion (36) hémisphérique par surmoulage.

8. Appareil selon l'une des revendications précédentes **caractérisé en ce que** ladite source lumineuse (4) comprend une pluralité de diodes électroluminescentes (4) réparties de façon régulière autour dudit guide de lumière (5).

9. Appareil selon l'une des revendications précédentes, **caractérisé en ce que** lesdits moyens de distribution (53) comprennent au moins une pente périphérique (531, 533) destinée au renvoi de lumière vers la périphérie du guide de lumière (5).

10. Appareil selon l'une des revendications précédentes, **caractérisé en ce que** lesdits moyens de distribution (53) comprennent au moins une pente intermédiaire (532) destinée au renvoi de lumière vers une zone intermédiaire du guide de lumière (5).

11. Appareil selon l'une des revendications précédentes, **caractérisé en ce que** ladite tête de massage (31) comprend des moyens de réflexion (35) permettant un renvoi de lumière vers ladite zone (Z) de contact.

12. Appareil selon l'une des revendications précédentes **caractérisé en ce que** ladite au moins une tête de soin (9) est détachable dudit corps (2).

13. Appareil selon la revendication précédente **caractérisé en ce qu'**il comprend au moins deux têtes de soin différentes interchangeables sur le corps (2).

## Patentansprüche

1. Behandlungsvorrichtung (1) für die Haut, umfassend:
- einen Körper (2) mit ein Gehäuse (21, 22), das eine Greifzone bildet,
- Stromversorgungsmittel (23), die in dem Körper angeordnet sind,
- mindestens ein Behandlungskopf (9) mit Massagemitteln (31)
- Antriebsmittel (26, 31') der Massagemittel (31), die von einem Elektromotor (24) betrieben werden, der mit den Stromversorgungsmitteln (23) verbunden ist,
- mindestens eine Lichtquelle (4),
- mindestens einen Lichtleiter (5), der eine Projektionsfläche (51) zum Empfangen von Licht aufweist, das von der mindestens einen Lichtquelle (4) emittiert wird, und zum Projizieren mindestens eines Lichtstrahls gezielt auf einen Bereich auf der Haut,
**dadurch gekennzeichnet, dass** der mindestens eine Lichtleiter (5) eine der Projektionsfläche (51) gegenüberliegende Fläche (52) umfasst, wobei die gegenüberliegende Fläche (52) Verteilungsmittel (53) zum Verteilen des Lichts entlang der Projektionsfläche (51) umfasst, und dass mindestens ein Massagemittel (31) eine Kontaktzone (Z) mit der zu massierenden Haut aufweist, wobei die Zone (Z) zur Lichtdurchlässigkeit bestimmt ist und aus einem Material vom thermoplastischen Elastomertyp (TPE) mit einer Lichtdurchlässigkeit zwischen 0,8 und 0,9 für ein Licht mit einer Wellenlänge von 590 nm oder 630 nm besteht.

2. Vorrichtung nach Anspruch 1, **dadurch gekennzeichnet, dass** das Material eine Härte zwischen 30 und 70 Shore A aufweist.

3. Vorrichtung nach einem der vorstehenden Ansprüche, **dadurch gekennzeichnet, dass** die Kontaktzone (Z) einen unpolierten Oberflächenzustand mit einer arithmetischen Rauheit Ra im Bereich von 0,5 µm bis 1 µm aufweist.

4. Vorrichtung nach einem der vorstehenden Ansprüche, **dadurch gekennzeichnet, dass** die Massagemittel (31) mindestens einen Massagekopf (31) umfassen, der dazu bestimmt ist, entlang mindestens einer vertikalen Drehachse (A1, A2, A3) gedreht zu werden, die senkrecht zur Projektionsfläche (51) verläuft.

5. Vorrichtung nach dem vorstehenden Anspruch, **dadurch gekennzeichnet, dass** der Massagekopf (31) einen halbkugelförmigen Abschnitt (36) aufweist.

6. Vorrichtung nach Anspruch 4, **dadurch gekennzeichnet, dass** der Massagekopf (31) eine Abdeckung (32) umfasst, welche die Kontaktzone (Z) definiert.

7. Vorrichtung nach dem vorstehenden Anspruch, **dadurch gekennzeichnet, dass** die Abdeckung (32) durch Umspritzen an dem hemisphärischen Abschnitt (36) befestigt wird.

8. Vorrichtung nach einem der vorstehenden Ansprüche, **dadurch gekennzeichnet, dass** die Lichtquelle (4) eine Vielzahl von lichtemittierenden Dioden (4) umfasst, die gleichmäßig um den Lichtleiter (5) herum verteilt sind.

9. Vorrichtung nach einem der vorstehenden Ansprüche, **dadurch gekennzeichnet, dass** die Verteilungsmittel (53) mindestens eine Umfangsneigung (531, 533) zum Umleiten von Licht zum Umfang des Lichtleiters (5) umfassen.

10. Vorrichtung nach einem der vorstehenden Ansprüche, **dadurch gekennzeichnet, dass** die Verteilungsmittel (53) mindestens eine Zwischenneigung (532) zum Umleiten von Licht in eine Zwischenzone des Lichtleiters (5) umfassen.

11. Vorrichtung nach einem der vorstehenden Ansprüche, **dadurch gekennzeichnet, dass** der Massagekopf (31) Reflexionsmittel (35) umfasst, die ein Umleiten von Licht in die Kontaktzone (Z) ermöglichen.

12. Vorrichtung nach einem der vorstehenden Ansprüche, **dadurch gekennzeichnet, dass** der mindestens eine Behandlungskopf (9) vom Körper (2) abnehmbar ist.

13. Vorrichtung nach dem vorstehenden Anspruch, **dadurch gekennzeichnet, dass** sie auf dem Körper (2) mindestens zwei verschiedene Behandlungsköpfe umfasst, die austauschbar sind.

## Claims

1. Method Skin treatment device (1) comprising:
- a body (2) comprising a housing (21, 22) forming a gripping area,
- electrical supply means (23) arranged in the body,
- at least one treatment head (9) comprising massage means (31)
- means (26, 31') for manoeuvring the massage means (31) actuated by an electric motor (24) which is connected to the electrical supply means (23),
- at least one light source (4),
- at least one light guide (5) having a projection surface (51) configured to receive the light emitted by the at least one light source (4) and to project at least one light beam toward an area on the skin,
**characterised in that** said at least one light guide (5) comprises a face (52) opposite the projection surface (51), the opposite face (52) comprising distribution means (53) configured to distribute the light along the projection surface (51), and **in that** at least one massage means (31) has an area (Z) for contact with the skin to be massaged, the area (Z) being configured to transmit the light and consisting of a thermoplastic elastomer material (TPE) having a light transmission of between 0.8 and 0.9 for light with a wavelength of 590 nm or 630 nm.

2. Device according to claim 1, **characterised in that** the material has a hardness of between 30 and 70 Shore A.

3. Device according to one of the preceding claims, **characterised in that** the contact area (Z) has an unpolished surface finish with an arithmetic roughness Ra of order 0.5 µm to 1 µm.

4. Device according to one of the preceding claims, **characterised in that** the massage means (31) comprise at least one massage head (31) configured to be moved in rotation according to at least one vertical axis of rotation (A1, A2, A3) which is perpendicular to the projection surface (51).

5. Device according to the preceding claim, **characterised in that** the massage head (31) has a hemispheric portion (36).

6. Device according to claim 4, **characterised in that** the massage head (31) comprises a cover (32) defining the contact area (Z).

7. Device according to the preceding claim, **characterised in that** said cover (32) is fixed to the hemispheric portion (36) by overmoulding.

8. Device according to one of the preceding claims, **characterised in that** the light source (4) comprises a plurality of light-emitting diodes (4) distributed regularly around the light guide (5).

9. Device according to one of the preceding claims, **characterised in that** the distribution means (53) comprise at least one peripheral slope (531, 533) configured to reflect the light toward the periphery of the light guide (5).

10. Device according to one of the preceding claims, **characterised in that** the distribution means (53) comprise at least one intermediate slope (532) configured to reflect the light toward an intermediate area of the light guide (5).

11. Device according to one of the preceding claims, **characterised in that** the massage head (31) comprises reflection means (35) permitting reflecting the light toward the contact area (Z).

12. Device according to one of the preceding claims, **characterised in that** the at least one treatment head (9) is detachable from the body (2).

13. Device according to the preceding claim, **characterised in that** it comprises at least two different treatment heads, interchangeable on the body (2).
